# FASCICULE DE BREVET EUROPEEN

(11) **EP 3 157 472 B1**
(45) Date de publication et mention de la délivrance du brevet: **16.05.2018**
(21) Numéro de dépôt: 15732867.5
(22) Date de dépôt: 11.06.2015
(51) Int. Cl.: A61F 2/52, A61F 2/50

(54) **PROTHÈSE MAMMAIRE EXTERNE**
EXTERNE BRUSTPROTHESE
EXTERNAL BREAST PROSTHESIS

(30) Priorité: 23.06.2014 FR 1455791
(43) Date de publication de la demande: 26.04.2017
(73) Titulaire: New-Team, 31620 Bouloc (FR)
(72) Inventeur: VALDISERRA, Christine, 33000 Bordeaux (FR); COMTE NÉE SANCHEZ, Léonarda, 31620 Bouloc (FR)
(74) Mandataire: IP Trust
(86) Numéro de dépôt international: PCT/FR2015/051554
(87) Numéro de publication internationale: WO 2015/197938

(56) Documents cités:
- EP-A1- 0 791 345
- BR-U- 6 801 634
- US-A- 2 851 692
- US-A- 3 811 133
- US-A- 4 364 880
- US-A- 5 458 635
- US-A1- 2005 197 698

## Description

L'invention concerne une prothèse mammaire externe, et plus particulièrement une telle prothèse, non fonctionnelle, destinée à remplacer temporairement un sein ayant subi une ablation et dont l'aspect et le comportement dynamique se rapproche le plus possible du sein original.

Les prothèses mammaires externes sont généralement utilisées après une mastectomie, après cicatrisation et avant une reconstruction chirurgicale de la poitrine, soit pendant une période de deux ans en général. Une prothèse mammaire externe peut être réalisée sur mesure pour une patiente donnée, ce qui implique en général un coût important, ou bien être choisie dans un assortiment de prothèses standards, plus économiques. Cependant, dans ce cas, certains ajustements, tels que le poids de la prothèse qui doit être sensiblement égal au poids du sein restant (dans le cas d'une mastectomie unilatérale) ne sont pas réalisés et peuvent à terme engendrer des complications telles que des attitudes scoliotiques ou générer un inconfort pour la patiente.

On connait, par exemple du document US 3,811,133 une prothèse mammaire externe comportant une enveloppe creuse en matière synthétique, par exemple en vinyle souple en forme de sein, un rembourrage fibreux et un élément pondéral variable constitué de granules de métal, par exemple du plomb liées dans un bloc de matière synthétique ou dans un contenant en feutre cousu. Ce poids est placé au plus près du torse de la patiente, fixé ou immobilisé sur une paroi arrière de l'enveloppe plaquée contre le torse de la patiente. Ce type de prothèse, qui s'apparente aux prothèses sur mesure présente néanmoins de nombreux inconvénients. Par exemple, le rembourrage utilisé ne permet pas à la prothèse de présenter un comportement dynamique comparable à celui d'un sein naturel ce qui peut être particulièrement visible lors de l'utilisation de la prothèse avec des vêtements légers tels qu'un soutien-gorge souple. US 2 851 692 A, qui est considéré comme l'état de la technique le plus proche, décrit également une prothèse mammaire externe comprenant une enveloppe en matériau souple présentant une forme de sein dont une face postérieure est adaptée pour être plaquée contre le torse d'une personne, et définissant une cavité interne renfermant un rembourrage incorporant une pluralité d'évidements contenant des poids.

La présente invention vise donc à fournir une prothèse mammaire externe qui ne présente pas les inconvénients des prothèses connues de la technique antérieure.

En particulier, l'invention vise à fournir une telle prothèse dont le poids est réglable par l'utilisatrice.

L'invention vise également une telle prothèse qui présente un comportement dynamique plus proche d'un sein naturel.

L'invention vise en outre une telle prothèse dont le couple de porte-à-faux soit ajustable.

L'invention vise encore une telle prothèse dont le coût de réalisation soit proche de celui d'une prothèse standard tout en offrant le confort et l'esthétique d'une prothèse sur mesure.

Pour ce faire, l'invention concerne une prothèse mammaire externe, comprenant une enveloppe en matériau souple présentant une forme de sein, ladite enveloppe définissant une cavité interne fermée par un opercule dont une face, dite face externe, est adaptée pour être plaquée contre le torse d'une personne, ladite cavité renfermant un rembourrage et un élément pondéral destiné à ajuster le poids de la prothèse, caractérisée en ce que l'élément pondéral est réalisé sous forme d'une pluralité d'éléments de lest distribués le long d'un axe sagittal de la prothèse sensiblement orthogonal au plan de l'opercule.

Dans la présente description, on utilise les termes classiquement employés dans le domaine médical en physiologie pour définir les différentes orientations dans l'espace par rapport au corps d'un patient, en l'occurrence les plans (frontal, sagittal, horizontal) ou axes (axe sagittal ou antéro-postérieur orthogonal au plan frontal ; axe transversal orthogonal au plan sagittal et axe vertical orthogonal au plan horizontal). Ces termes prenant pour référence le corps d'un patient, le plan horizontal par rapport au corps peut être vertical par rapport à la surface terrestre si le corps est en position couchée.

En utilisant une pluralité d'éléments de lest distribués dans l'axe sagittal de la personne portant la prothèse, il est possible non seulement d'ajuster le poids total de la prothèse pour le faire correspondre à celui du sein manquant ou de l'autre sein, mais également de modifier son couple de porte-à-faux de manière à conserver une sensation plus proche de l'original dans les mouvements du corps, et ce, même si ce couple est compensé par le soutien-gorge. De même, le déplacement et/ou la modification de forme de la prothèse au cours de ces mouvements sont plus proches de ceux d'un sein naturel dont le comportement dynamique dépend de la répartition de sa masse dans toutes les directions, notamment selon l'axe sagittal.

Avantageusement et selon l'invention, la cavité interne présente une forme intérieure dont le contour selon une coupe par un plan frontal est curviligne, notamment un contour sensiblement circulaire, elliptique ou ovale. Par exemple, la cavité interne présente une forme sensiblement tronconique, de hauteur orientée selon un axe sagittal. Cette forme permet d'utiliser des éléments de lest présentant une forme de disques (ou de discoïdes) placés orthogonalement à l'axe sagittal de la prothèse. Il est également possible de choisir une cavité interne présentant une forme de calotte sphérique qui présente des avantages similaires.

Avantageusement et selon l'invention, la cavité est adaptée pour contenir de trois à cinq éléments de lest de tailles étagées, décroissantes de l'opercule à l'extrémité distale de la cavité. Afin d'obtenir une bonne répartition du couple de porte-à-faux de la prothèse, il est avantageux de prévoir que la cavité puisse contenir suffisamment d'éléments de lest et que ces éléments puissent être ordonnés dans la cavité. Les inventeurs ont constaté qu'un résultat satisfaisant est obtenu avec trois à cinq éléments dont les tailles (en l'occurrence les diamètres) sont étagées pour que leur bord vienne au contact ou au voisinage proche de la paroi de la cavité, selon un ordre prédéterminé (le plus petit à l'extrémité distale de la cavité).

Avantageusement et selon l'invention, pour chaque taille d'élément de lest, il existe une pluralité d'éléments de lest présentant des poids différents. De cette manière il est possible pour l'utilisatrice de réaliser la répartition optimale du couple de porte-à-faux en choisissant le poids de chaque élément de lest en fonction de sa position le long de l'axe sagittal de la prothèse. Il est ainsi possible de jouer au moyen de l'élasticité de l'enveloppe et par un choix approprié des éléments de lest, sur la forme générale du sein dans un plan vertical sagittal, par exemple obtenir un sein "lourd" en choisissant des éléments de lest de poids plus important en position distale ou bien un sein pommé en plaçant les éléments de lest les plus lourds en position proximale.

Avantageusement et selon un premier mode de réalisation de l'invention, les éléments de lest sont des disques pondéreux insérés dans des fentes parallèles ménagées dans un rembourrage en mousse ajusté à la forme de la cavité interne. Grâce à l'utilisation d'un rembourrage en mousse, de préférence très souple, muni de fentes parallèles et orthogonales à l'axe sagittal de la prothèse, chaque section du rembourrage, de part et d'autre d'une fente, peut se déplacer par rapport aux sections adjacentes sous l'effet de sollicitations statiques ou dynamiques liées au poids du disque pondéral insérés dans la fente. Ces disques peuvent être des disques métalliques de densité différente ou des disques de matière synthétique chargés par des particules de densités différentes ou par des particules de même densité en proportions différentes. De manière préférentielle, les disques pondéraux sont immobilisés dans leur fente, soit par des reliefs adaptés, soit par la forme de la fente qui se referme au-dessus du disque une fois celui-ci inséré.

Avantageusement et selon un deuxième mode préférentiel de réalisation de l'invention, les éléments de lest sont des coussinets de lestage de forme sensiblement lenticulaire dont le plan médian principal est sensiblement parallèle au plan de l'opercule. En utilisant ainsi des coussinets lenticulaires, c'est-à-dire présentant une épaisseur tangible, de préférence correspondant à une fraction de la profondeur de la cavité, il est possible de se dispenser du rembourrage en mousse du mode de réalisation précédent, l'épaisseur de chacun des coussinets contribuant au remplissage de la cavité de la prothèse. La taille (par exemple le diamètre pour un coussinet de forme circulaire) est adaptée, comme vu précédemment, pour venir au contact de la paroi interne de la cavité en fonction du rang du coussinet sur l'axe sagittal de la prothèse.

Avantageusement et selon l'invention, chaque coussinet de lestage comprend une paroi externe en forme de sachet souple remplie d'une charge déformable. Chaque sachet peut être formé par la réunion de deux feuilles de matière synthétique souple, par exemple en élastomère, soudées entre elles par leurs bords en regard selon un contour sensiblement circulaire, elliptique ou ovale en fonction de la forme de la cavité de la prothèse (orthogonalement à l'axe sagittal de celle-ci). À l'intérieur de chaque sachet, une charge de remplissage permet de donner sa forme lenticulaire au sachet. La charge est de préférence plastique, c'est-à-dire déformable et présente une élasticité réduite ou nulle.

Avantageusement et selon l'invention, la charge déformable est constituée d'un solide divisé d'une masse volumique adaptée au poids recherché. Par exemple, la charge déformable peut être formée d'un granulat de matière solide, par exemple de microbilles d'un diamètre de 10 µm à 500 µm. De telles microbilles de verre peuvent présenter une masse volumique apparente allant de 0,2 kg/dm³ pour des billes creuses jusqu'à 1,5 kg/dm³ pour des billes pleines. Pour des coussinets de poids plus élevé, il est possible d'utiliser des microsphères de métal qui présentent des densités supérieures, ou des mélanges de différents types de granulats.

Avantageusement et selon l'invention, la charge déformable est constituée d'un gel polymère comportant une charge dont la densité est fonction du poids recherché. De manière préférentielle, pour améliorer la sensation tactile ressentie, il est possible d'intégrer la charge solide divisée dans un liant tel qu'un gel polymère pour obtenir une charge viscoélastique d'un seul tenant. La masse volumique de cette charge déformable est alors déterminée par la masse volumique du gel, celle de la charge solide et par la proportion de charge solide dans le gel.

Avantageusement et selon l'invention, les coussinets de lestage comportent des moyens de fixation placés sur un axe sagittal de la prothèse et adaptés pour rendre solidaires des faces en regard de deux coussinets adjacents. Ainsi chaque coussinet de lestage peut être rendu solidaire des coussinets adjacents tout en conservant une certaine mobilité apportée par le mouvement relatif des deux feuilles constituant les faces du sachet. De cette manière, l'ensemble des coussinets se comporte comme une masse viscoélastique souple possédant la capacité de s'incliner de part et d'autre de l'axe sagittal en fonction des mouvements du buste de l'utilisatrice, à l'instar d'un sein naturel.

Avantageusement et selon l'invention, la prothèse comporte en outre un disque de matière élastique adapté pour être placé entre l'opercule et les coussinets de lestage pour maintenir ces derniers en compression. En fonction du poids et du comportement dynamique recherchés, il n'est pas nécessaire d'employer tous les coussinets d'une prothèse. Par exemple, il est possible de n'utiliser que trois coussinets de lestage sur les cinq que peut accueillir l'enveloppe de la prothèse. Il est alors possible d'utiliser un tampon de comblement en mousse synthétique placé entre les coussinets et l'opercule formant la face arrière de la prothèse afin de maintenir les coussinets en place, avec un minimum de compression pour éviter tout déplacement des coussinets de lestage qui pourrait se traduire par une forme disgracieuse de la prothèse.

Avantageusement et selon l'invention, l'enveloppe est réalisée en mousse thermoplastique thermoformée, notamment choisie parmi les mousses de polyoléfines, en particulier en mousse de polyéthylène thermoformée. Outre les matériaux synthétiques souples couramment employés pour réaliser des prothèses externes, tels que silicone, latex ou vinyle, l'invention propose d'utiliser une enveloppe en polyéthylène, préférablement en polyéthylène basse densité pour sa souplesse. Encore plus avantageusement, l'enveloppe peut être réalisée en mousse de polyéthylène qui présente l'avantage d'être très légère et de pouvoir être thermoformée à la forme désirée par des opérations de production plus économiques que celles utilisées pour les autres matériaux. En outre, une telle mousse de polyéthylène thermoformée présente l'avantage d'offrir une surface d'un toucher très doux, bien plus proche de la sensation tactile de la peau que les autres matières.

Avantageusement et selon l'invention, l'enveloppe est teintée dans la masse et comporte un mamelon rapporté. De plus, la mousse de polyéthylène présente l'avantage de pouvoir être teintée dans la masse et de présenter au moins sur une face un aspect de "peau". En outre, afin de prendre en compte les différentes teintes de carnation, il est possible de réaliser un mamelon et son aréole attenante dans une autre teinte de mousse et de le rapporter par exemple par collage sur l'enveloppe. Avantageusement, dans ce cas, la mousse utilisée peut présenter une densité supérieure pour offrir une meilleure résistance à l'abrasion dans cette zone fortement sollicitée au frottement.

Avantageusement et selon l'invention, l'enveloppe comporte au moins une ouverture de la cavité permettant l'introduction des éléments de lest. L'ouverture de la cavité est préférentiellement réalisée par séparation entre le bord de l'opercule et celui de l'enveloppe, sur une partie supérieure du contour de celle-ci selon le plan frontal de la prothèse. Par exemple, l'une des deux surfaces en regard du bord de l'opercule ou de l'enveloppe comporte une bande adhésive double face, le cas échéant réutilisable, permettant de refermer la cavité après introduction des éléments de lest.

Avantageusement et selon l'invention, l'opercule comporte, sur sa face externe, une bande de fixation recouverte d'une pluralité de films protecteurs détachables juxtaposés de manière à permettre le choix d'au moins une zone de collage sur le torse. Quoique la prothèse mammaire externe selon l'invention soit prévue pour être portée à l'intérieur d'un soutien-gorge, il est cependant préférable qu'elle soit fixée au torse de l'utilisatrice. À cette fin, l'invention prévoit que l'opercule, sur sa face externe en contact avec le torse, comporte une bande adhésive, par exemple une bande adhésive double face biocompatible résistante à l'eau et autres produits courants de toilette. La bande adhésive est fixée d'origine sur la face externe de l'opercule et comporte en regard du torse de l'utilisatrice une pluralité de films protecteurs susceptibles d'être ôtés pour assurer un collage sur le torse. Les films protecteurs sont disposés de manière à permettre à l'utilisatrice de choisir les zones de collage de la prothèse en fonction de la disposition de zones de sensibilité particulières (par exemple cicatrices, etc.)

L'invention concerne également une prothèse mammaire externe caractérisée en combinaison par tout ou partie des caractéristiques mentionnées ci-dessus ou ci-après.

D'autres buts, caractéristiques et avantages de l'invention apparaîtront au vu de la description qui va suivre et des dessins annexés dans lesquels :
- la figure 1 est une vue en coupe schématique par un plan sagittal médian d'une prothèse mammaire externe selon un mode de réalisation préférentiel de l'invention,
- la figure 2 est une vue en coupe selon le même plan que la figure 1 d'une prothèse selon un autre mode de réalisation,
- la figure 3 est une vue selon l'axe vertical d'une poitrine d'une personne couchée comprenant un sein naturel et une prothèse selon l'invention, illustrant le mécanisme du réalisme statique et dynamique de la prothèse, et
- la figure 4 est une vue en perspective de trois quart arrière montrant la face externe de l'opercule et la bande adhésive permettant de fixer la prothèse selon l'invention sur le torse d'une personne.

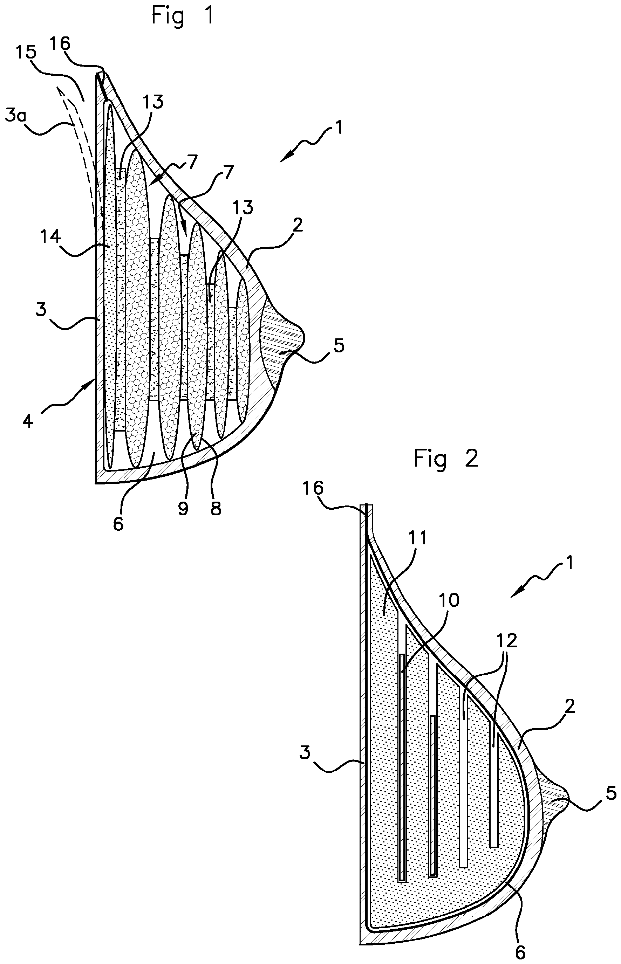

La prothèse 1 mammaire externe représentée à la figure 1 comporte une enveloppe 2 en forme de sein réalisée dans un matériau souple et flexible, préférentiellement en mousse de polyéthylène de basse densité d'une épaisseur variant entre 3 mm et 10 mm.

L'enveloppe 2 définit une cavité 6 interne fermée par un opercule 3 sensiblement plan. L'opercule 3 peut être réalisé d'un seul tenant avec l'enveloppe 2 ou bien être collé ou soudé sur le contour de l'enveloppe 2 pour fermer la cavité 6. Dans un cas comme dans l'autre, une ouverture 15 de la cavité est ménagée dans la partie supérieure du contour de l'enveloppe pour permettre l'introduction d'éléments de lest et de rembourrage dans la cavité. L'ouverture 15 est obtenue en séparant une partie (représentée en tirets à la figure 1) de l'opercule 3 du contour de l'enveloppe 2. Elle peut être refermée par collage au moyen d'une fermeture 16 adhésive réutilisable.

Dans le mode de réalisation préférentiel illustré à la figure 1, la cavité 6 contient cinq éléments de lest sous la forme de coussinets 7 de lestage de forme lenticulaire et de tailles étagées. Les coussinets 7 sont disposés de manière à ce que leur plan médian soit sensiblement orthogonal à l'axe sagittal de la prothèse 1, parallèlement à l'opercule 3. Chaque coussinet 7 est d'une taille (en l'occurrence d'un diamètre) adaptée pour correspondre au diamètre interne de la cavité 6 en fonction de sa position sur l'axe sagittal. Par exemple, le coussinet 7 placé à l'extrémité distale de la cavité 6 présente le plus petit diamètre.

Les coussinets 7 de lestage comportent une paroi externe formant un sachet 8 souple contenant une charge 9 déformable. Le sachet 8 peut être réalisé par la jonction, selon leur bord, de deux disques de matière synthétique souple, par exemple en élastomère. Avant jonction complète, le sachet 8 est rempli avec une charge 9 qui lui donne son épaisseur et donc sa forme lenticulaire. La charge 9 peut être constituée d'une poudre ou de granules, par exemple de billes de verre, qui, en combinaison avec l'élasticité du sachet 8, donnent une consistance viscoélastique au coussinet 7, consistance s'apparentant à la consistance naturelle du sein. Alternativement, les sachets 8 peuvent être remplis par un gel polymère, par exemple un hydrogel silicone seul ou servant de liant à une charge granulaire.

Selon l'invention, pour chaque taille de coussinet 7, il existe une pluralité de coussinets 7 présentant un poids différent afin de permettre, pour un poids total donné correspondant au poids du sein remplacé, de disposer de plusieurs solutions de répartition du poids le long de l'axe sagittal. Pour une même taille de coussinet, les différents poids peuvent être obtenus en jouant sur la densité du gel, sur la densité (réelle ou apparente) de la charge granulaire, sur la proportion de charge dans le gel, etc. Par exemple une charge granulaire formée de microbilles de verre creuses peut présenter une densité apparente allant de 0,2 à 1,5. Des densités supérieures peuvent être obtenues avec des microbilles d'une autre matière, par exemple des microbilles métalliques. En fonction de la taille et donc du volume du coussinet, toutes les gammes de poids peuvent être obtenues en réalisant le cas échéant des mélanges de différentes charges. Bien entendu, les différentes charges ne sont pas limitées aux microbilles citées mais peuvent comprendre des talcs ou tout autre type de charge connu de l'homme du métier.

Les coussinets 7 comportent en outre des moyens de fixation entre coussinets adjacents. Dans l'exemple représenté à la figure 1, ces moyens de fixation sont des pastilles adhésives 13, disposées selon l'axe sagittal. Ces pastilles adhésives 13 sont bien entendu réutilisables pour permettre des changements de coussinets à la volonté de l'utilisatrice. Ces pastilles adhésives présentent un diamètre inférieur au diamètre des coussinets qu'elles relient de manière à laisser libre une couronne de chaque face du sachet 8 afin de permettre un déplacement latéral de chaque coussinet par rapport aux coussinets adjacents par déformation de cette couronne du sachet.

Afin de maintenir les coussinets 7 de lestage dans la cavité 6, un tampon 14 de comblement, en matière élastique de forme appropriée, par exemple un cylindre en mousse élastique, termine l'empilage des coussinets et sert de ressort entre l'opercule 3 et le plus grand coussinet afin de conserver les coussinets en compression.

L'enveloppe 2 de la prothèse 1 peut être réalisée d'un seul tenant. Cependant, selon l'invention, il peut être préférable de prévoir de rapporter un insert 5 formant le mamelon et la zone aréolaire périphérique pour d'une part permettre un changement de teinte adapté, le mamelon et l'aréole étant généralement d'une carnation plus soutenue que le reste du sein et d'autre part utiliser une matière plus résistante à l'abrasion, compte tenu que cette zone est beaucoup plus sujette à des sollicitations de frottement. Le mamelon rapporté peut être fixé par collage ou bien par coopération de formes conjuguées lors du thermoformage de l'enveloppe 2.

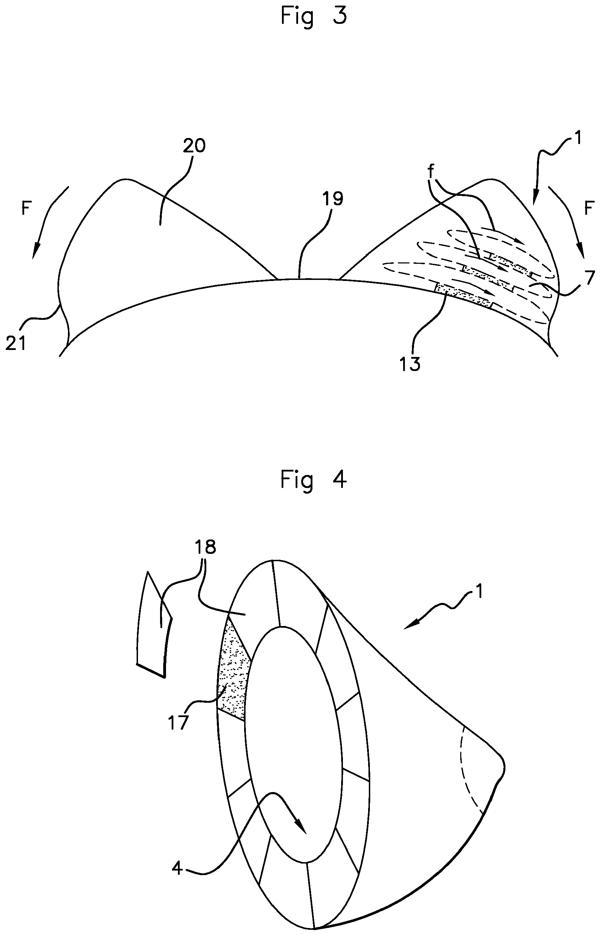

On se réfère à la figure 3 pour détailler l'un des avantages de la prothèse selon l'invention. On a représenté sur cette figure, en position couchée, une poitrine comprenant un sein naturel 20 et une prothèse 1 vue selon un axe correspondant à l'axe vertical du corps. En position couchée, le sein naturel 20 a tendance à se déplacer vers l'extérieur par rapport au torse 19 (mouvement illustré par la flèche F) et à former un galbe 21. Dans les prothèses de la technique antérieure, le rembourrage de densité constante et la place du poids au plus près du torse font que la prothèse reste immobile et rigide. Pour la prothèse 1 selon l'invention, les inventeurs ont constaté que l'ensemble des coussinets 7 se comporte comme une masse viscoélastique souple grâce à la répartition du poids le long de l'axe sagittal et à la déformation de chaque coussinet 7 dont les faces antérieure et postérieure du sachet glissent latéralement l'une par rapport à l'autre tout en étant maintenues respectivement par la pastille adhésive 13 à la face correspondante du coussinet adjacent. Ce glissement relatif des coussinets 7, illustré par les flèches f sur la figure 3, entraine une déformation de l'enveloppe 2 selon une flèche F similaire à celle du sein naturel et provoque un galbe analogue. De même, de nombreux mouvements de la poitrine sont rendus possibles par la prothèse selon l'invention et le sentiment de confort de l'utilisatrice est renforcé.

Un autre mode de réalisation de la prothèse 1 est illustré à la figure 2. Dans ce mode de réalisation, la cavité 6 de l'enveloppe 2 est remplie par un rembourrage 11 en mousse ajusté à la forme de la cavité. Les éléments de lest se présentent sous la forme de disques 10 pondéreux insérés dans des fentes 12 ménagées dans le rembourrage 11. Chaque fente 12 présente, en fonction de son rang sur l'axe sagittal, un contour correspondant au contour du disque 10 correspondant à ce rang, à l'exception de la zone d'insertion du disque dans la partie supérieure du rembourrage. La taille des disques 10 est adaptée pour laisser une épaisseur de mousse tout autour du disque de manière à ne pas influer sur la sensation tactile. La mousse formant le rembourrage 11 est suffisamment souple pour permettre un mouvement latéral relatif des disques 10 les uns par rapport aux autres, autorisant ainsi une dynamique de la prothèse analogue à celle obtenue avec les coussinets. Pour chaque taille de disque 10, il est fourni une pluralité de disques 10 de poids différent permettant ainsi de moduler le couple s'exerçant sur l'enveloppe 2 en fonction du porte-à-faux de la prothèse. Dans ce mode de réalisation, l'enveloppe 2 comporte également une ouverture permettant l'insertion du rembourrage 11 et des disques 10 dans la cavité, ouverture refermée par une fermeture adhésive 16. L'enveloppe 2 peut également comporter un mamelon 5 rapporté.

Quel que soit le mode de réalisation de la prothèse selon l'invention, celle-ci comporte des moyens de fixation de la prothèse sur le torse d'une utilisatrice. Ces moyens sont représentés à la figure 4 et comportent, sur la face externe 4 de l'opercule 3 une bande de fixation 17, par exemple composée d'un adhésif double face biocompatible. Cette bande de fixation 17 présente une forme adaptée pour suivre le contour de l'opercule 3 et une largeur suffisante pour permettre une fixation adaptée de la prothèse sur le torse de l'utilisatrice. Avantageusement, cette bande de fixation est recouverte d'une pluralité de films protecteurs 18 détachables juxtaposés recouvrant toute la surface de la bande de fixation 17. Cette disposition avantageuse permet à l'utilisatrice de choisir la zone de collage de la prothèse sur son torse de manière à éviter des zones de sensibilité particulière telles que cicatrices, etc. La bande de fixation 17 peut être avantageusement résistante à l'eau et aux produits de toilette courants de manière à pouvoir être portée en permanence.

Bien entendu, cette description est donnée à titre d'exemple illustratif uniquement et l'homme du métier pourra y apporter de nombreuses modifications sans sortir de la portée de l'invention, comme par exemple modifier l'emplacement de l'ouverture 15 de l'enveloppe 2 en toute zone du contour de l'opercule 3 ou même au centre de celui-ci. De même, le tampon 14 de comblement dans le mode de réalisation de la figure 1 pourrait être placé de manière équivalente à l'extrémité distale de la cavité 6 tout en assurant sa fonction de ressort.

## Revendications

1. - Prothèse (1) mammaire externe, comprenant une enveloppe (2) en matériau souple présentant une forme de sein, ladite enveloppe définissant une cavité (6) interne fermée par un opercule (3) dont une face, dite face externe (4), est adaptée pour être plaquée contre le torse d'une personne, ladite cavité renfermant un rembourrage et un élément pondéral destiné à ajuster le poids de la prothèse, l'élément pondéral étant réalisé sous forme d'une pluralité d'éléments de lest (7, 10), **caractérisée en ce que** ladite pluralité d'éléments de lest (7, 10) sont distribués le long d'un axe sagittal de la prothèse sensiblement orthogonal au plan de l'opercule.

2. - Prothèse selon la revendication 1, **caractérisée en ce que** la cavité (6) interne présente une forme intérieure dont le contour selon une coupe par un plan frontal est curviligne, notamment un contour sensiblement circulaire, elliptique ou ovale.

3. - Prothèse selon l'une des revendications 1 ou 2, **caractérisée en ce que** la cavité (6) est adaptée pour contenir de trois à cinq éléments de lest (7, 10) de tailles étagées, décroissantes de l'opercule (3) à l'extrémité distale de la cavité.

4. - Prothèse selon la revendication 3, **caractérisée en ce que**, pour chaque taille d'élément de lest (7, 10), il existe une pluralité d'éléments de lest présentant des poids différents.

5. - Prothèse selon l'une des revendications 1 à 4, **caractérisée en ce que** les éléments de lest sont des disques (10) pondéreux insérés dans des fentes (12) parallèles ménagées dans un rembourrage (11) en mousse ajusté à la forme de la cavité (6) interne.

6. - Prothèse selon l'une des revendications 1 à 4, **caractérisée en ce que** les éléments de lest sont des coussinets (7) de lestage de forme sensiblement lenticulaire dont le plan médian principal est sensiblement parallèle au plan de l'opercule (3).

7. - Prothèse selon la revendication 6, **caractérisée en ce que** chaque coussinet (7) de lestage comprend une paroi externe en forme de sachet (8) souple remplie d'une charge (9) déformable.

8. - Prothèse selon la revendication 7, **caractérisée en ce que** la charge (9) déformable est constituée d'un solide divisé d'une masse volumique adaptée au poids recherché.

9. - Prothèse selon la revendication 7, **caractérisée en ce que** la charge (9) déformable est constituée d'un gel polymère comportant une charge dont la densité est fonction du poids recherché.

10. - Prothèse selon l'une des revendications 6 à 9, **caractérisée en ce que** les coussinets (7) de lestage comportent des moyens (13) de fixation placés sur un axe sagittal de la prothèse et adaptés pour rendre solidaires des faces en regard de deux coussinets adjacents.

11. - Prothèse selon l'une des revendications 6 à 10, **caractérisée en ce qu'**elle comporte en outre un tampon (14) de comblement en matière élastique adapté pour être placé entre l'opercule (3) et les coussinets (7) de lestage pour maintenir ces derniers en compression.

12. - Prothèse selon l'une des revendications 1 à 11, **caractérisée en ce que** l'enveloppe (2) est réalisée en mousse thermoplastique thermoformée, notamment choisie parmi les mousses de polyoléfines, en particulier en mousse de polyéthylène thermoformée.

13. - Prothèse selon l'une des revendications 1 à 12, **caractérisée en ce que** l'enveloppe (2) est teintée dans la masse et comporte un mamelon (5) rapporté.

14. - Prothèse selon l'une des revendications 1 à 13, **caractérisée en ce que** l'enveloppe (2) comporte au moins une ouverture (15) de la cavité (6) permettant l'introduction des éléments (7; 10) de lest.

15. - Prothèse selon l'une des revendications 1 à 14, **caractérisée en ce que** l'opercule (3) comporte, sur sa face externe (4), une bande de fixation (17) recouverte d'une pluralité de films (18) protecteurs détachables juxtaposés de manière à permettre le choix d'au moins une zone de collage sur le torse.

## Patentansprüche

1. Externe Brustprothese (1) bestehend aus einer Hülle (2) aus weichem Material in Form einer Brust, wobei besagte Hülle einen inneren Hohlraum (6) abgrenzt, der durch eine Abdeckung (3) geschlossen wird, deren eine Seite, die sogenannte äußere Seite (4), so gestaltet ist, dass sie sich an den Oberkörper einer Person anschmiegt, wobei besagter Hohlraum eine Füllung und ein gewichtsregulierendes Element zur Angleichung des Prothesengewichts umschließt, wobei das gewichtsregulierende Element aus einer Vielzahl von Beschwerungsselementen (7, 10) besteht, **dadurch gekennzeichnet, dass** besagte Vielzahl von Beschwerungselementen (7, 10) auf einer sagittalen Achse der Prothese im Wesentlichen orthogonal zur Ebene der Abdeckung verteilt ist.

2. Prothese nach Anspruch 1, **dadurch gekennzeichnet, dass** der innere Hohlraum (6) eine Innenform aufweist, deren Kontur nach einem Zuschnitt über eine Frontalebene bogenförmig und im Wesentlichen rund, elliptisch oder oval ist.

3. Prothese nach einem der Ansprüche 1 oder 2, **dadurch gekennzeichnet, dass** der Hohlraum (6) so gestaltet ist, dass er am distalen Ende 3 bis 5 Beschwerungselemente (7, 10) in abgestuften, von der Abdeckung (3) des Hohlraums aus kleiner werdenden Größen enthalten kann.

4. Prothese nach Anspruch 3, **dadurch gekennzeichnet, dass** es für jede Größe eines Beschwerungselements (7, 10) eine Vielzahl von Beschwerungselementen mit unterschiedlichen Gewichten gibt.

5. Prothese nach einem der Ansprüche 1 bis 4, **dadurch gekennzeichnet, dass** die Beschwerungselemente gewichtsregulierende Scheiben (10) sind, welche in parallele Schlitze (12), die in eine, an die Form des inneren Hohlraums (6) angepasste Füllung (11) aus Schaumstoff eingearbeitet wurden, geschoben werden.

6. Prothese nach einem der Ansprüche 1 bis 4, **dadurch gekennzeichnet, dass** es sich bei den Beschwerungselementen um Beschwerungskissen (7) in einer im Wesentlichen linsenförmigen Form handelt, deren Hauptmittelebene im Wesentlichen parallel zur Ebene der Abdeckung (3) verläuft.

7. Prothese nach Anspruch 6, **dadurch gekennzeichnet, dass** jedes Beschwerungskissen (7) eine Außenwand in Form eines weichen, mit einem verformbaren Füllstoff (9) gefüllten Beutels (8) aufweist.

8. Prothese nach Anspruch 7, **dadurch gekennzeichnet, dass** der verformbare Füllstoff (9) aus feinteiligen Feststoffen mit einer Massendichte besteht, die an das gesuchte Gewicht angepasst wird.

9. Prothese nach Anspruch 7, **dadurch gekennzeichnet, dass** der verformbare Füllstoff (9) aus einem Polymergel mit einem Füllstoff besteht, dessen Dichte vom gesuchten Gewicht abhängt.

10. Prothese nach einem der Ansprüche 6 bis 9, **dadurch gekennzeichnet, dass** die Beschwerungskissen (7) Befestigungsmittel (13) umfassen, welche auf einer sagittalen Achse der Prothese angeordnet sind und so ausgebildet wurden, dass sie die gegenüberliegenden Flächen zweier benachbarter Kissen fest miteinander verbinden.

11. Prothese nach einem der Ansprüche 6 bis 10, **dadurch gekennzeichnet, dass** sie des Weiteren eine Fülllage (14) aus elastischem Material umfasst, welche so ausgebildet ist, dass sie zwischen der Abdeckung (3) und den Beschwerungskissen (7) positioniert werden kann, um den Pressdruck der letzteren aufrecht zu halten.

12. Prothese nach einem der Ansprüche 1 bis 11, **dadurch gekennzeichnet, dass** die Hülle (2) aus thermoplastischem, thermogeformtem Schaumstoff hergestellt ist, der insbesondere aus Poliolefin-Schaumstoffen ausgewählt wird, und insbesondere aus thermogeformtem Polyethylen.

13. Prothese nach einem der Ansprüche 1 bis 12, **dadurch gekennzeichnet, dass** die Hülle (2) massegefärbt ist und eine angesetzte Mamille (5) aufweist.

14. Prothese nach einem der Ansprüche 1 bis 13, **dadurch gekennzeichnet, dass** die Hülle (2) mindestens eine Öffnung (15) zum Hohlraum (6) hin umfasst, welche das Hineinschieben von Beschwerungselementen (7, 10) ermöglicht.

15. Prothese nach einem der Ansprüche 1 bis 14, **dadurch gekennzeichnet, dass** die Abdeckung (3) an ihrer Außenseite (4) einen Befestigungsstreifen (17) aufweist, der mit einer Vielzahl von loslösbaren Trennfolien (18) bedeckt ist, die so nebeneinander angeordnet wurden, dass mindestens ein Klebebereich auf dem Oberkörper ausgewählt werden kann.

## Claims

1. An external breast prosthesis (1) comprising a shell (2) in a flexible material having a breast shape, said shell defining an internal cavity (6) formed by a lid (3), a face of which, called external face (4), is adapted so as to be pressed against the chest of a person, said cavity containing a filling and a weighted element intended for adjusting the weight of the prosthesis, **characterized in that** the weighted element is made as a plurality of ballast elements (7, 10) distributed along a sagittal axis of the prosthesis substantially orthogonal to the plane of the lid.

2. The prosthesis according to claim 1, **characterized in that** the internal cavity (6) has an interior shape, the contour of which along a section through a front plane is curvilinear, notably a substantially circular, elliptical or oval contour.

3. The prosthesis according to one of claims 1 or 2, **characterized in that** the cavity (6) is adapted for containing from three to five ballast elements (7, 10) of staged sizes, decreasing from the lid (3) to the distal end of the cavity.

4. The prosthesis according to claim 3, **characterized in that**, for each size of a ballast element (7, 10), there exists a plurality of ballast elements having different weights.

5. The prosthesis according to one of claims 1 to 4, **characterized in that** the ballast elements are weighted discs (10) inserted into parallel slots (12) made in a foam filling (11) adjusted to the shape of the internal cavity (6).

6. The prosthesis according to one of claims 1 to 4, **characterized in that** the ballast elements are ballast pads (7) with a substantially lenticular shape, the main median plane of which is substantially parallel to the plane of the lid (3).

7. The prosthesis according to claim 6, **characterized in that** each ballast pad (7) comprises an external flexible wall with a pouch shape (8) filled with a deformable load (9).

8. The prosthesis according to claim 7, **characterized in that** the deformable load (9) consists of a divided solid with a specific density adapted to the sought weight.

9. The prosthesis according to claim 7, **characterized in that** the deformable load (9) consists of a polymeric gel including a load, the density of which depends on the sought weight.

10. The prosthesis according to one of claims 6 to 9, **characterized in that** the ballast pads (7) include attachment means (13) placed on a sagittal axis of the prosthesis and adapted for making faces secured to each other facing two adjacent pads.

11. The prosthesis according to one of claims 6 to 10, **characterized in that** it further includes a filling buffer (14) in elastic material suitable for being placed between the lid (3) and the ballast pads (7) in order to maintain the latter in compression.

12. The prosthesis according to one of claims 1 to 11, **characterized in that** the shell (2) is made in a thermoformed thermoplastic foam, notably selected from among polyolefin foams, in particular in thermoformed polyethylene foam.

13. The prosthesis according to one of claims 1 to 12, **characterized in that** the shell (2) is tinted in the bulk and includes an added nipple (5).

14. The prosthesis according to one of claims 1 to 13, **characterized in that** the shell (2) includes at least one opening (15) of the cavity (6) allowing introduction of the ballast elements (7; 10).

15. The prosthesis according to one of claims 1 to 14, **characterized in that** the lid (3) includes, on its external face (4) an attachment strip (17) covered with a plurality of juxtaposed detachable protective films (18) so as to allow the selection of at least one area for adhesive bonding on the chest.
